Europäisches Patentamt

European Patent Office

Office européen des brevets

(19)

(11) Veröffentlichungsnummer: **0 008 636**
**B1**

(12) **EUROPÄISCHE PATENTSCHRIFT**

(45) Veröffentlichungstag der Patentschrift: **23.06.82**

(51) Int. Cl.³: **A 61 M 5/30**

(21) Anmeldenummer: **79102383.1**

(22) Anmeldetag: **11.07.79**

(54) Impfpistole.

(30) Priorität: **22.07.78 DE 2832252**

(43) Veröffentlichungstag der Anmeldung:
**19.03.80 Patentblatt 80/6**

(45) Bekanntmachung des Hinweises auf die Patenterteilung:
**23.06.82 Patentblatt 82/25**

(84) Benannte Vertragsstaaten:
**DE FR GB**

(56) Entgegenhaltungen:
**DE - B - 1 944 006**
**US - A - 2 653 602**
**US - A - 3 945 379**

(73) Patentinhaber: **HOECHST AKTIENGESELLSCHAFT**
**Postfach 80 03 20**
**D-6230 Frankfurt/Main 80 (DE)**

(72) Erfinder: **Zimmermann, Josef**
**Auf der Krautweide 11**
**D-6231 Sulzbach (DE)**

Courier Press, Leamington Spa, England.

Impfpistole

Die Erfindung betrifft eine Impfpistole für medizinische Zwecke, die eine Ventilklappe aufweist, um den Gasdruck für den Antriebsmotor der Impfstoffpumpe nahezu konstant zu halten.

Nach der deutschen Patentschrift 1 922 569 ist eine Impfpistole gemäß dem Oberbegriff des Patentanspruchs 1 bekannt, bei der die Impfstoffpumpe durch einen mit Gas betriebenen Motor angetrieben wird. Nachteilig ist, daß bei unterschiedlichem Gasdruck, z.B. hervorgerufen durch unterschiedlichen Füllstand in der Gasvorratsflasche, der Motor unterschiedliche Kräfte auf die Impfstoffpumpe überträgt, so daß sich die Einschußtiefe des Impfstoffes in das Gewebe mit dem Gasdruck ändert. Aus medizinischen Gründen sollen die Einschußdrücke an der Mündung der Impfstoffdüse ca. 270—300 bar nicht übersteigen. Frisch gefüllte Gasflaschen erzeugen jedoch Einschußdrücke bis ca. 750 bar, wodurch der Impfstoff in nicht gewünschte Gewebetiefen gelangt.

Nach der US-Patentschrift 3 945 379 ist eine Impfpistole bekannt, bei der die Impfstoffpumpe ebenfalls durch einen mit Gas betriebenen Motor angetrieben wird. Zwischen Gasvorratsbehälter und Antriebsmotor ist eine Kammer angeordnet, die zum Antriebsmotor hin durch ein Ventil verschlossen ist. Dieses Ventil, das durch eine Feder geschlossen gehalten wird, öffnet, sobald der Gasdruck den Federdruck überwindet. Durch diese Maßnahme soll gewährleistet sein, daß der Antriebsmotor bei jedem Impfschuß nahezu die gleiche Kraft auf die Impfstoffpumpe überträgt. Nachteilig ist die aufwendige Konstruktion der Kammer und des Ventils, wobei letzteres besonders störanfällig ist, wenn sich die Impfpistole bei Serienimpfungen durch die ständige Gasentspannung abkühlt.

Hier will die Erfindung Abhilfe schaffen. Die Impfpistole soll mit einfachen, störunanfälligen Mitteln so verbessert werden, daß der Einschußdruck an der Mündung der Imstoffdüse über den gesamten Arbeitsbereich der Gasvorratsflasche zwischen 270 und 300 bar liegt.

Die Erfindung, wie sie in den Ansprüchen gekennzeichnet ist, löst die Aufgabe dadurch, daß zwischen Absperrventil und Anschluß für den Vorratsbehälter eine Kammer angeordnet ist, in der sich eine Ventilklappe befindet, die einen hohlen Schaft aufweist, der in einem Teil der Kammer ständig geführt und mit radialen Bohrungen versehen ist wobei der den Schaft führende und mit seinem Ende mit dem Absperrventil verbundene Teil der Kammer nach Entleerung der Kammer durch die Ventilklappe geschlossen und darauf durch Druckausgleich auf beiden Seiten der Ventilklappe dieser Teil der Kammer wieder geöffnet werden kann.

Um nach erfolgtem Schuß den Druckausgleich auf beiden Seiten der Ventilklappe herbeizuführen, kann die Klappe mit einer Leckeinrichtung in Form einer engen Bohrung oder einer Rinne versehen sein, so daß allmählich Gas von der Vorratsflasche zum Absperrventil gelangen kann. Der Druckausgleich kann jedoch auch über eine Umgehungsleitung, in der gegebenenfalls ein Ventil angeordnet sein kann, erfolgen. Die Ventilklappe kann jedoch auch mechanisch, z. B. mit einer Abdrückvorrichtung, geöffnet werden. Diese Abdrückvorrichtung ist in dem Teil der Kammer angeordnet, in dem der Schaft der Ventilklappe geführt wird.

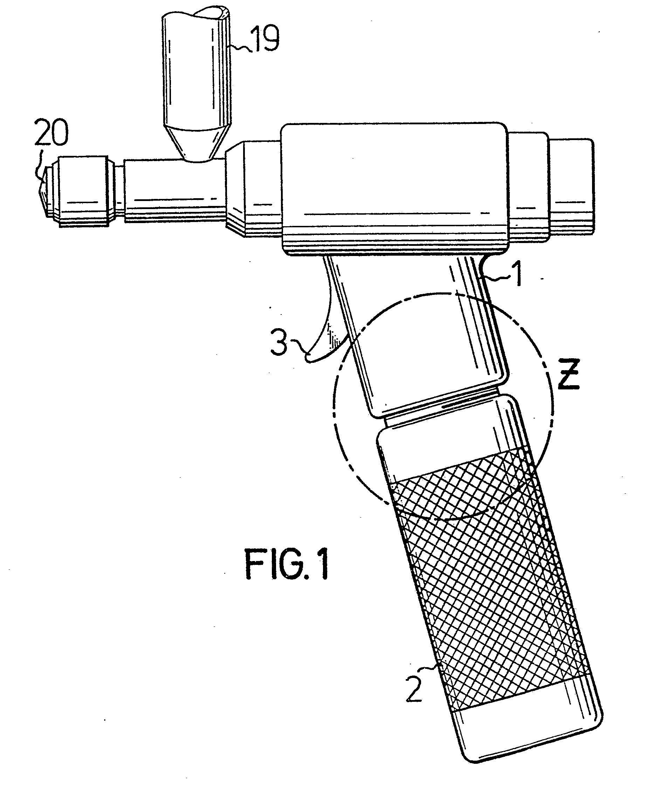

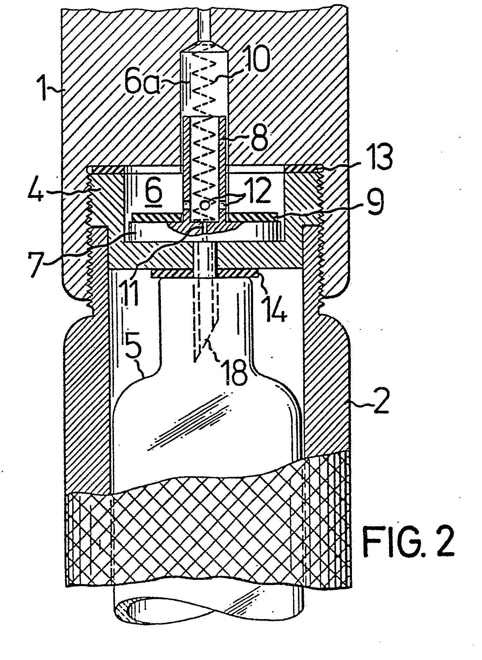

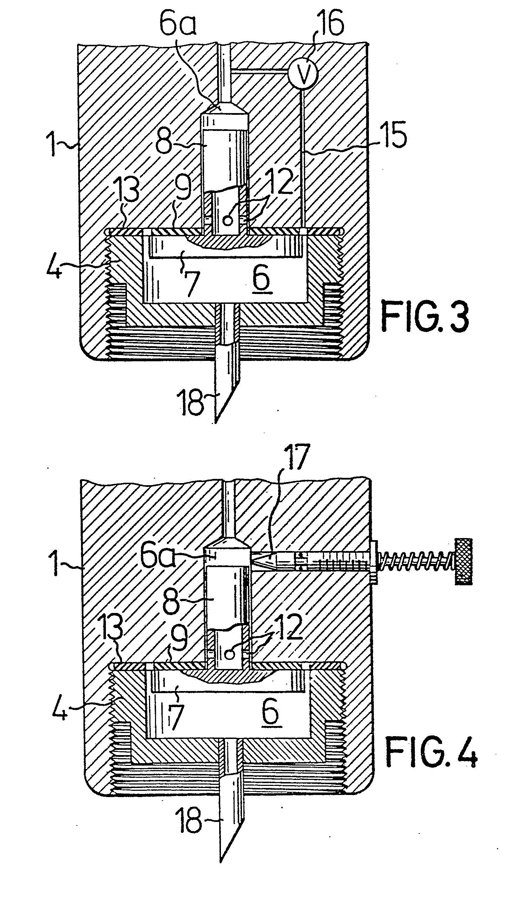

Im Folgenden wird die Erfindung anhand von lediglich einen Ausführungsweg darstellenden Figuren näher erläutert. Es zeigt Fig. 1 die Impfpistole, Fig. 2 die Einzelheit Z aus Figur 1 geschnitten, Fig. 3 die Umgehungseinrichtung für die Ventilklappe und Fig. 4 die Abdrückvorrichtung für die Ventilklappe.

Der Pistolengriff besteht im wesentlichen aus dem Flansch 1 und dem in den Flansch 1 einschraubbaren Griff 2. Im Flansch 1 ist das Absperrventil 3 für das Triebgas des Antriebsmotors und der Anschluß 4 für den Vorratsbehälter 5 für das Gas angeordnet. Der Anschluß 4 ist so ausgebildet, daß er mit dem Flansch 1 eine Kammer 6, 6a bildet. In dieser Kammer 6, 6a ist eine Ventilklappe 7 mit hohlem Schaft 8 angeordnet. Der Schaft 8 wird in Teil 6a der Kammer 6, 6a geführt. Die Ventilklappe 7 ist ferner auf der dem Flansch 1 zugewandten Seite mit einer Dichtung 9 versehen und kann eine Leckeinrichtung 11 aufweisen. Im Innern des Schaftes 8 kann eine Druckfeder 10 angeordnet sein. Damit beim Schließen der Ventilklappe 7 das Gas in Richtung Motor entweichen kann, ist der Schaft 8 mit Bohrungen 12 versehen. Mittels Dichtung 13 wird der Anschluß 4 gegen den Flansch 1 und mittels Dichtung 14 der Vorratsbehälter 5 gegen den Anschluß 4 abgedichtet. Die Leckeinrichtung 11 — hier eine Bohrung — ist so dimensioniert, daß während des Impfschusses kein nennenswerter Druckausgleich auf beiden Seiten der Ventilklappe 7 stattfinden kann. Der Griff 2 kann einen Vorratsbehälter 5 für Gas, wie dargestellt, enthalten. Der Vorratsbehälter 5 kann ohne weiteres durch einen Adapter für andere Gasbehälter ersetzt werden. 18 deutet die Stichverbindung für den Vorratsbehälter 5 an. 19 ist der Impfstoffbehälter und 20 die Impfstoffdüse.

Um die Kammer 6, 6a nach erfolgtem Impfschuß wieder vollständig mit Gas zu füllen, ist es erforderlich, die Ventilklappe 7 zu öffnen. Dazu ist ein Druckausgleich auf beiden Seiten der Ventilklappe 7 erforderlich. Das kann wie bereits erwähnt durch die Bohrung 12 geschehen. Es ist jedoch auch möglich, die Dichtung 9 mit einer kleinen Rinne zu versehen. Teil 6a der Kammer 6, 6a kann auch mit einer Umgehungsleitung 15, in die ein Absperrventil 16 eingebaut ist, verbunden sein. In dem Teil 6a der Kammer 6, 6a kann aber auch eine mechanische Abdrückvorrichtung 17 zum Öffnen der Ventilklappe 7 hineinragen.

Vor dem Impfschuß ist die Kammer 6, 6a mit Gas, das unter dem Druck des Vorratsbehälters 5 steht, gefüllt. Beim Öffnen des Absperrventils 3 strömt das Gas aus Kammer 6, 6a zum Antriebsmotor der Impfstoffpumpe (nicht dargestellt). Durch das aus dem Vorratsbehälter 5 nachströmende Gas wird die Ventilklappe 7 mit ihrer Dichtung 9 gegen den Flansch 1 gedrückt, wodurch ein Nachströmen von Gas aus dem Vorratsbehälter 5 zum Antriebsmotor unterbunden wird. Durch einen Druckausgleich auf beiden Seiten der Ventilklappe 7 wird dieselbe geöffnet und die Impfpistole für den nächsten Schuß vorbereitet.

## Patentansprüche

1. Impfpistole mit einer Impfstoffkolbenpumpe und einem mit Gas betriebenen Antriebsmotor für die Impfstoffpumpe, der in einem an einem Ende der Impfstoffpumpe ankuppelbaren Gehäuse angeordnet ist und über ein Absperrventil (3) mit einem Vorratsbehälter (5) für Triebgas in Verbindung steht, dessen Anschluß (4) im Pistolengriff untergebracht ist, dadurch gekennzeichnet, daß zwischen Absperrventil (3) und dem Anschluß (4) für den Vorratsbehälter (5) eine Kammer (6, 6a) angeordnet ist, in der sich eine Ventilklappe (7) befindet, die einen hohlen Schaft (8) aufweist, der in einem Teil (6a) der Kammer (6, 6a) ständig geführt und mit radialen Bohrungen (12) versehen ist wobei der den Schaft (8) führende und mit seinem Ende mit dem Absperrventil (3) verbundene Teil (6a) der Kammer (6, 6a) nach Entleerung der Kammer (6, 6a) durch die Ventilklappe (7) geschlossen und darauf durch Druckausgleich auf beiden Seiten der Ventilklappe (7) dieser Teil (6a) der Kammer (6, 6a) wieder geöffnet werden kann.

2. Impfpistole nach Anspruch 1, dadurch gekennzeichnet, daß die Ventilklappe (7) eine Leckeinrichtung (11) aufweist.

3. Impfpistole nach Anspruch 1, dadurch gekennzeichnet, daß die Kammer (6, 6a) eine Umgehungsleitung (15) aufweist.

4. Impfpistole nach Anspruch 1, dadurch gekennzeichnet, daß der Teil (6a) der Kammer (6, 6a), der den Schaft (8) führt, mit einer mechanischen Abdrückvorrichtung (17) versehen ist.

## Claims

1. Vaccination gun having a vaccine piston pump, drive motor means for driving the vaccine pump, said drive motor means being operated by a propellant gas and arranged in a housing, which can be coupled at one end of the vaccine pump, and connected via a locking valve (3) with a store container for the propellant gas, the connection (4) of which is housed within the gun grip, characterized by that a chamber (6, 6a) is arranged between locking valve (3) and connection (4) for the store container (5), which chamber houses a flap-valve (7) provided with a hollow shaft (8) extending into part (6a) of chamber (6, 6a) and being provided with radial borings (12), the part (6a) of chamber (6, 6a) which is connected by its end with locking valve (3) and into which extends shaft (8) is closed by flap-valve (7) after discharging chamber (6, 6a) and then said part (6a) of chamber (6, 6a) may be opened again by equalizing pressure on both sides of flap-valve (7).

2. A vaccination gun as claimed in claim 1, wherein the flap-valve (7) is provided with a leakage arrangement (11).

3. A vaccination gun as claimed in claim 1, wherein the chamber (6, 6a) is provided with a bypass line (15).

4. A vaccination gun as claimed in claim 1, wherein part (6a) of chamber (6, 6a), comprising shaft (8), is provided with a mechanical trigger device (17).

## Revendications

1. Pistolet d'inoculation muni d'une pompe à vaccin à piston et d'un moteur fonctionnant au gaz pour l'entraînement de la pompe à vaccin, moteur qui est monté dans un carter pouvant être accouplé à une extrémité de la pompe à vaccin et qui est relié par l'intermédiaire d'une soupape d'arret (3) à un réservoir de gaz d'actionnement (5) dont le raccordement (4) est logé dans la crosse du pistolet, caractérisé en ce qu'entre la soupape d'arrêt (3) et le raccordement (4) destiné au réservoir (5) est disposée une chambre (6, 6a) dans laquelle se trouve un clapet (7) qui présente une tige creuse (8) guidée constamment dans une partie (6a) de la chambre (6, 6a) et munie de perforations radiales (12), et en ce qu'une fois la chambre (6, 6a) vidée, la partie (6a) de la chambre (6, 6a) qui guide la tige (8) et qui est reliée par son extrémité à la soupape d'arrêt (3) peut être fermée par le clapet (7) et qu'ensuite, cette partie (6a) de la chambre (6, 6a) peut être ouverte à nouveau par égalisation de la pression entre les deux côtés du clapet (7).

2. Pistolet d'inoculation selon la revendication 1, caractérisé en ce que le clapet (7) présente un dispositif de fuite (11).

3. Pistolet d'inoculation selon la revendication 1, caractérisé en ce que la chambre (6, 6a) comprend un conduit de dérivation (15).

4. Pistolet d'inoculation selon la revendication 1, caractérisé en ce que la partie (6a) de la chambre (6, 6a) qui guide la tige (8) est munie d'un dispositif mécanique de poussée (17).

0 008 636

FIG.1

FIG. 2

FIG.3

FIG.4